# EUROPEAN PATENT APPLICATION

(11) **EP 4 477 135 A1**
(43) Date of publication of application: **18.12.2024**
(21) Application number: 23178625.2
(22) Date of filing: 12.06.2023
(51) Int. Cl.: A61B 5/00, A61B 5/11, A46B 15/00, A61C 17/22

(54) **SYSTEM AND METHOD FOR DETECTING TREMORS**

(71) Applicant: Koninklijke Philips N.V., 5656 AG Eindhoven (NL)
(72) Inventor: HIWALE, Sujitkumar, Eindhoven (NL); JOHNSON, Mark Thomas, Eindhoven (NL); PERRONE, Antonio Luigi, 5656AG Eindhoven (NL); VAN DEN DUNGEN, Wilhelmus Andreas Marinus Arnoldus Maria, Eindhoven (NL); GERHARDT, Lutz Christian, Eindhoven (NL)
(74) Representative: Philips Intellectual Property & Standards

(57) **Abstract**

A hand-held personal care device has an actuator for implementing a personal care function which generates a vibration. A sensor signal is obtained which depends on motion in particular vibration of the personal care device. Tremor vibrations are detected from the sensor signal in order to detect tremor. By detecting the tremor vibrations based on the way they modulate (e.g., dampen or otherwise influence) existing driven vibrations of the personal care function, it has been found that a simple low cost sensor may be used. In particular, the sensor may comprise an actuator current sensor or a simple single axis vibration (i.e., motion) sensor.

## Description

### FIELD OF THE INVENTION

This invention relates to the detection of tremors, and in particular using a device which has a vibratory personal care function.

### BACKGROUND OF THE INVENTION

Tremor is defined as a rhythmical, involuntary oscillatory movement of a body part. Tremors are result of involuntary alternating contractions of reciprocally innervated muscles. Tremors are the most common movement disorder symptom, especially in the elderly population.

Tremors are likely to hamper many basic self-care activities, which require a good amount of motor coordination such as brushing, shaving, bathing etc. These activities are often referred as Activities of Daily Living (ADLs). This can lead to a major change in daily routine, absenteeism from work or even psychological issues.

Essential tremor (ET) is the most common cause of action tremor, with an estimated prevalence worldwide of 1 percent overall and approximately 5 percent in adults over the age of 60 years. The incidence of ET increases with age, although childhood and early adulthood presentations do occur, especially when ET is familial.

In context of oral health, poor motor coordination can lead to poor brushing quality, which can lead to suboptimal oral hygiene, which ultimately can lead to oral diseases. Therefore, it would be important in such cases to provide some means to minimize the physical disability due to tremors so that a person can perform the required physical activity with minimum discomfort.

The most common method for diagnosis of tremors is analysis of a patient's history and thorough physical examination along with targeted neurological assessment. The physical examination for tremor involves assessment of different characteristic of tremors such as affected body parts, position of body part, relation with body movements (at rest; during action) and frequency of tremors. Such characterization helps in grouping tremors according to their pathophysiology and aetiology, which in turn is highly relevant for choosing the most promising treatment option for tremors. The characteristic of different tremors and how they can be detected are discussed below.

### Orthostatic tremor

This is a postural tremor in the torso and lower limbs while standing, and it may also occur in the upper limbs. It is suppressed by walking. The tremor is high frequency (14 to 20 Hz) and synchronous among ipsilateral and contralateral muscles.

### Physiologic tremor

This is a predominantly bilateral, symmetrical action tremor. The tremor is again high frequency (10 to 12 Hz), with known causes (e.g., medications, hyperthyroidism, hypoglycemia).

### Essential tremor

This usually presents as a bilateral postural tremor of the hands in a frequency range 6 to 12Hz, followed by a kinetic and resting component.

### Parkinsonism Tremor

This is predominantly at rest, and is asymmetrical. Usually it does not produce head tremor. It has a frequency range 4 to 6 Hz.

### Cerebellar tremor

This is a postural, intention, or action tremor. It has a relatively low frequency in the range 3 to 4 Hz and is associated with ataxia and dysmetria.

### Writing tremor

This is not evident in other tasks requiring coordination, only during writing. It is considered a variant of focal hand dystonia (writer's cramp). It has no specific frequency range.

### Psychogenic tremor

This is an exclusion diagnosis. Symptoms vary in severity, depending on the subject's emotional state associated with stressful life events. Entrainment is a change in frequency of the tremor in adaptation to voluntary movements, such as a regular movement in the contralateral limb. It has not specific frequency range.

The physical assessment for tremors requires an in person visit to a physician's office, which may not be convenient, especially in the elderly population. Given the slow progression of disease, the disability could be missed during routine examination or could be quite significant by the time it is diagnosed by a physician especially given visits to physician's office are not so frequent. Another disadvantage is the subjective nature of examination, which makes it difficult to assess disease progression or to judge improvement after treatment.

An accelerometer-based technique has been proposed as a proxy to measure and quantify tremors. It is also possible to detect and classify generalized motor disorders (e.g., essential tremors or Parkinsonism tremors) using accelerometer signals obtained from a handheld device (e.g., smartphone) during normal physical activity. For daily use, a disadvantage of accelerometer-based methods comes from an obligation on the user to wear such a senor (e.g., a wrist band) all the time without fail. It is also associated with additional cost and may not be useful in elderly people due to the known issue of poor compliance (with any additional device) in this age group.

Tremors are treated based on the underlying cause/pathology of the tremors. A wide variety of treatment modalities are available for tremors, but treatment needs to be tailored for an individual, taking into account the objective measurement on tremor severity, and the degree of disability or overall impairment experienced by the patient.

As explained above, tremors develop rather slowly and it is difficult to detect them at early stages using conventional methods. There is a subjective element to physical assessment and there is no solution which fits well into a daily workflow. Disease progression trends are also difficult to obtain using conventional methods, which makes it difficult to assess disease progression or improvement after treatment.

There is therefore a need for an improved tremor detection approach.

### SUMMARY OF THE INVENTION

The invention is defined by the claims.

According to examples in accordance with an aspect of the invention, there is provided a hand-held personal care device comprising:
a handle;
an actuator for implementing a personal care function which generates a vibration;
a sensor for generating a sensor signal which depends on motion of the personal care device; and
a processing arrangement, configured to detect tremor vibrations from the sensor signal and thereby detect tremor,
wherein the sensor comprises an actuator current sensor or a single axis vibration sensor.

The invention provides a personal care device which generates a vibration. The invention is based on the recognition that when a user who suffers from tremor holds such a device, the lower frequency tremor vibrations are superposed with, or dampen, the vibrations generated by the actuator. The result is that tremor vibrations can be detected using a low cost sensor, and in particular more simply and reliably than detecting the tremor motion pattern. In particular, vibrating devices amplify the system response to the tremor on specific frequencies such as harmonics of the vibration function base frequency, due to an interaction between the function actuation and the tremor motion. These specific responses are due to the non-linearity of the system.

The motion sensed by the sensor at least includes a vibration.

One example is a single axis vibration sensor. Another example is an actuator current sensor. In this latter case, the tremor vibrations are detectable as they provide a load to the actuator which is detectable in the actuator drive current. Thus, the tremor vibrations induce a system response which influences the actuator current. The characteristic frequencies of those vibrations can thereby be detected from the actuator current.

In one example, based on a single axis vibration sensor, the sensor comprises a microphone. This provides a simple low cost sensing approach.

The processing arrangement is preferably configured to classify a detected tremor as one of a set of different types of tremor. The different tremor types for example have different frequency characteristics, but they may also have different temporal characteristics (i.e. amplitudes over time).

For example, a tremor may be characterized as orthostatic, physiologic, essential tremor, Parkinsonism or cerebellar based at least in part on the dominant tremor frequency.

The processing arrangement may be configured to determine a severity level of a detected tremor. This is based on a detected tremor amplitude and optionally duration information.

The processing arrangement is for example configured to determine a progression of a detected tremor over time.

In one arrangement, the processing arrangement comprises a band pass filter arrangement. This provides a simple circuit solution.

The band pass filter arrangement for example comprises a first set of band pass filters within an expected frequency range of the tremors and a set of further band pass filters in a frequency range of higher harmonics of the expected frequency range of the tremors. Thus, the filters of the first set select different tremor types, but the detection is improved using detection of harmonics as well.

The band pass filter arrangement for example further comprises a further blocking filter for blocking an operating frequency of the vibration generated by the actuator.

In another arrangement, the processing arrangement comprises a neural network. This enables more accurate detection, and may use the effect of the tremor vibration frequency and strength on the full frequency spectrum over a small period of time.

The neural network is for example supplied with a spectrogram of the sensor signal for a continuous range of frequencies up to at least double the personal care function vibration frequency. Thus, at least some harmonics are included in the spectrogram.

The device for example comprises a powered toothbrush or a shaver.

The invention also provides a method of detecting tremor, comprising:
receiving a sensor signal of an actuator current sensor or a single axis vibration sensor of a hand-held personal care device having an actuator for implementing a personal care function which generates a vibration; and
analyzing the sensor signal to detect tremor.

The invention also provides a computer program comprising computer program code means which is adapted, when said program is run on a computer, to implement the method defined above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows an example of an electric toothbrush which detect tremor in the user;
Fig. 2 shows a plot of a detection signal versus time, for a toothbrush held by a hand with a tremor for a manual brushing session;
Fig. 3 shows a plot of the detection signal versus time, for the toothbrush held by a hand without a tremor for a manual brushing session;
Fig. 4 shows a plot of detection signal versus time, for a toothbrush held by a hand with a tremor, with the electric toothbrush function turned on;
Fig. 5 shows a plot of detection signal versus time, for a toothbrush held by a hand without a tremor, with the electric toothbrush function turned on;
Fig. 6 shows FFT plots of FFT amplitude (y-axis) versus frequency, for a frequency range 0 to 8000Hz for a toothbrush held with different grips and with and without tremor;
Fig. 7 shows a first example of a processing arrangement, to detect tremor vibrations from the sensor signal; and
Fig. 8 shows a second example of a processing arrangement, to detect tremor vibrations from the sensor signal.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a hand-held personal care device which has an actuator for implementing a personal care function which generates a vibration. A sensor signal is obtained which depends on motion in particular vibration of the personal care device. Tremor vibrations are detected from the sensor signal in order to detect tremor. By detecting the tremor vibrations based on the way they modulate (e.g., dampen or otherwise influence) existing driven vibrations of the personal care function, it has been found that a simple low cost sensor may be used. In particular, the sensor may comprise an actuator current sensor or a simple single axis vibration (i.e., motion) sensor, a most simple example of which is a microphone.

The invention may be applied to any personal care device which generates a vibration when performing the personal care function. The personal care function may be a function for promoting health or for treating a medical condition or it may be part of a personal hygiene routine.

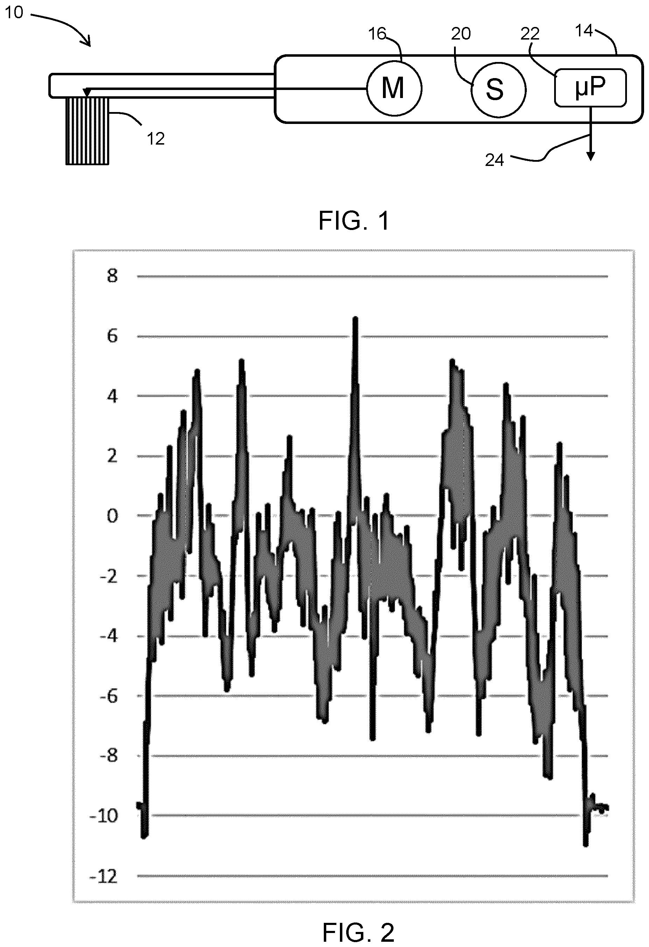

Fig. 1 shows an example of an electric toothbrush 10 having a driven head 12 and a handle 14, with an actuator 16 in the handle for driving the head. The actuator in one example is a motor and the head is driven to rotate, and this generates a vibration which is transmitted to the device body. A sensor 20 generates a sensor signal which depends on a vibration (or vibration pattern) of the toothbrush.

A processing arrangement, schematically shown as processor 22, detects tremor vibrations from the sensor signal and thereby detects tremor. An output 24 is generated which may indicate when tremor has arisen, or more preferably it may identify different types of tremor and monitor progression of tremor symptoms over time. These options are discussed below.

One option is that sensor comprises a motor current sensor. In this case, the tremor vibrations, which are applied to the handle by the user, act as a load to the motor and the change in load modulates the motor current, by which is meant dampen or otherwise influence the existing vibrations of the personal care function and thereby alter the current. Thus, the motor current may be interpreted to determine the presence of, and more preferably the type of, tremor of the subject holding the toothbrush 10. In contrast to full 3 axis motion based methods, another example is a single axis vibration sensor. A direction vector for the vibrations is not needed. Only the frequency and amplitude characteristics of the vibration are needed to enable the modulation created by the tremor of the user to be identified. The single axis vibration sensor may be implemented as a microphone.

Tremors can be identified from the sensor signal in general by detecting or filtering out characteristic frequency bands from the vibration frequencies of the toothbrush present in the sensor signal. An electric toothbrush for example operates with a rotation frequency around 70 to 80Hz, and a sonic toothbrush for example operates at around 260Hz, whereas the specific tremor frequencies lie in the range 3 to 20 Hz. Instead of filtering functions, power spectral analysis (using a FFT) and/or spectrograms may be employed.

A most basic implementation of the invention uses a sensor in the form of a single axis accelerometer. The head of a powered toothbrush and its handle are usually integrated with many sensors such as an inertial measurement unit, IMU, camera, pressure, pain sensors etc. The IMU sensor can be used to obtain an accelerometer signal during a brushing activity. In particular, a single axis of the accelerometer of the IMU can be used on a higher sampling frequency, optionally without low pass filtering. The IMU is normally used on lower speeds to detect 3D motion and position tracking.

The feasibility of the sensing approach described above has been demonstrated by simulating brushing sessions.

Fig. 2 shows a plot of the detection signal (e.g., single axis accelerometer signal) versus time, for a toothbrush held by a hand with a tremor, during a simulated brushing session without the electric toothbrush function turned on.

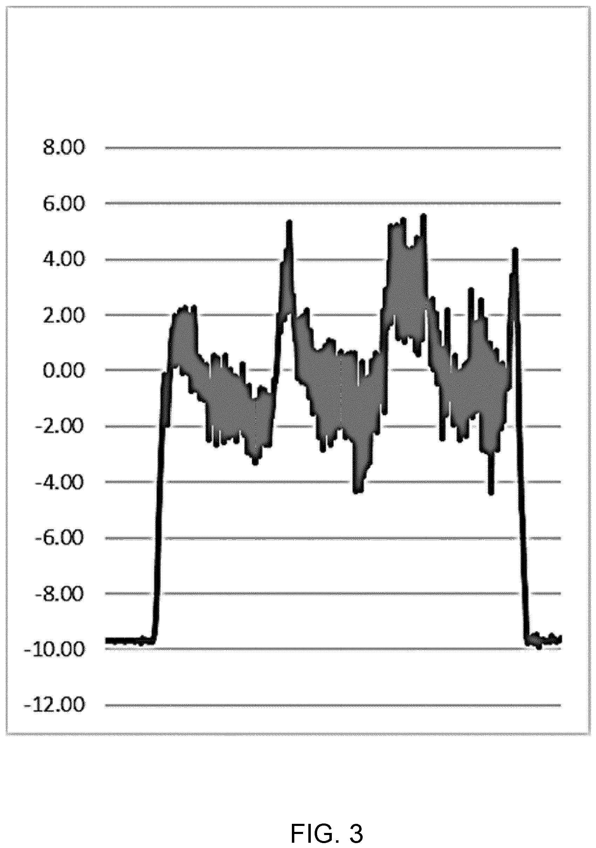

Fig. 3 shows a plot of the detection signal versus time, for the toothbrush held by a hand without a tremor, and again during a simulated brushing session without the electric toothbrush function turned on.

The peaks of the vibration motion imposed by the hand's tremor on the toothbrush movement can clearly be seen. The more frequent periodic peaks are clearly not present in Fig. 3. The only peaks present in Fig. 3 are peak due to the quadrant change during brushing and the associated switch between front and back teeth surfaces.

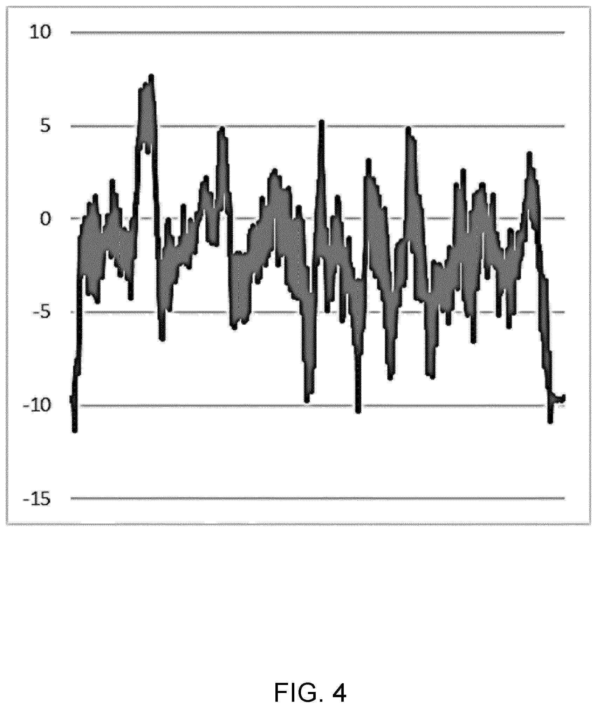

Fig. 4 shows a plot of detection signal (e.g. single axis accelerometer signal) versus time, for a toothbrush held by a hand with a tremor, during a simulated brushing session with the electric toothbrush function turned on.

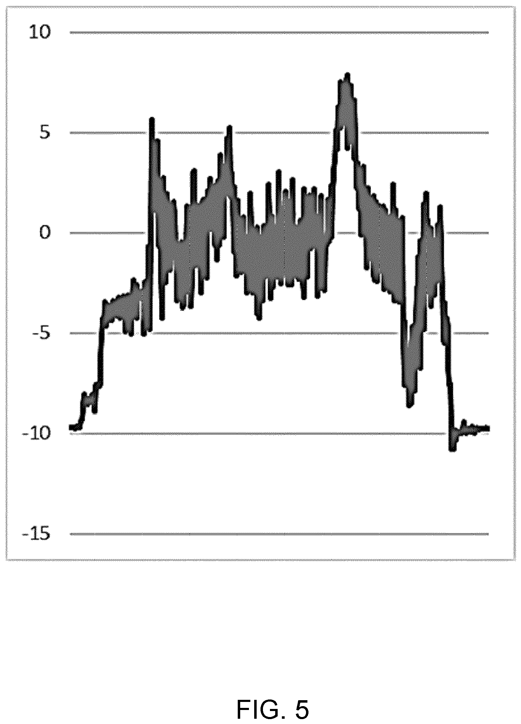

Fig. 5 shows a plot of detection signal (e.g. single axis accelerometer signal) versus time, for a toothbrush held by a hand without a tremor, during a simulated brushing session with the electric toothbrush function turned on.

The same differences arise between the plots, but there is the presence of a higher frequency oscillation resulting from the driven toothbrush function, compared to the plots of Figs 2 and 3.

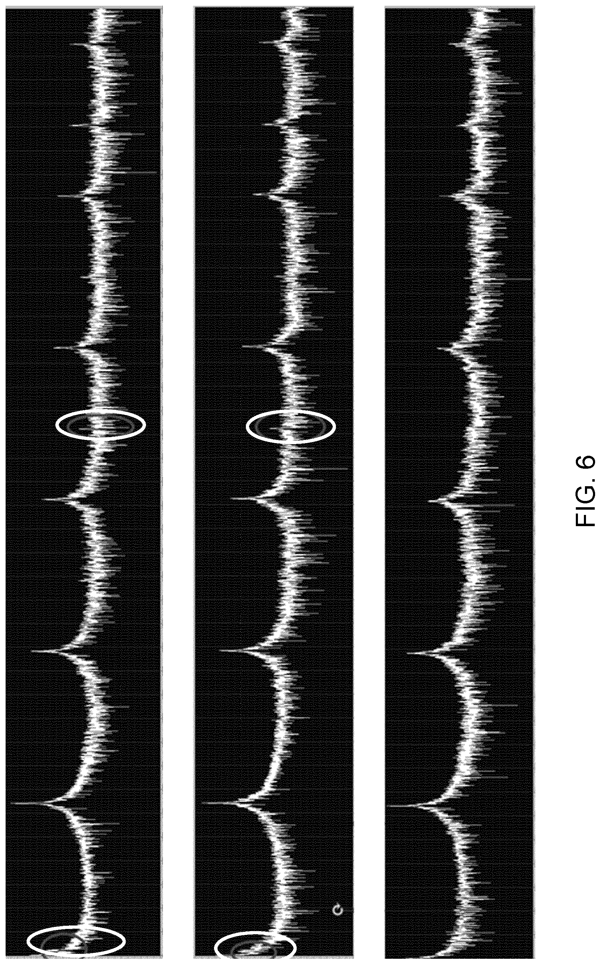

Fig. 6 shows FFT plot of FFT amplitude (y-axis) versus frequency, for a frequency range 0 to 8000Hz.

The top plot is for a toothbrush held with full grip and with a dynamic brushing style, with a tremor. The second plot is for a finger grip with a dynamic brushing style with a tremor. The bottom plot is for a full grip with a dynamic brushing style and no tremor.

Significant differences between the tremor and non-tremor plots arise at the lowest frequencies (i.e. the natural frequency of the tremor vibrations, 0 to 20Hz, as well as in a band of higher harmonics in the range 4400-4500 Hz. These areas are shown by rings. Both in full or palm grip and finger grip the specific tremor frequencies and harmonics can be seen. If the tremor is more severe, the amplitude of the specific tremor frequencies may also increase or change.

It can be seen therefore that tremor detection can be based on a frequency analysis.

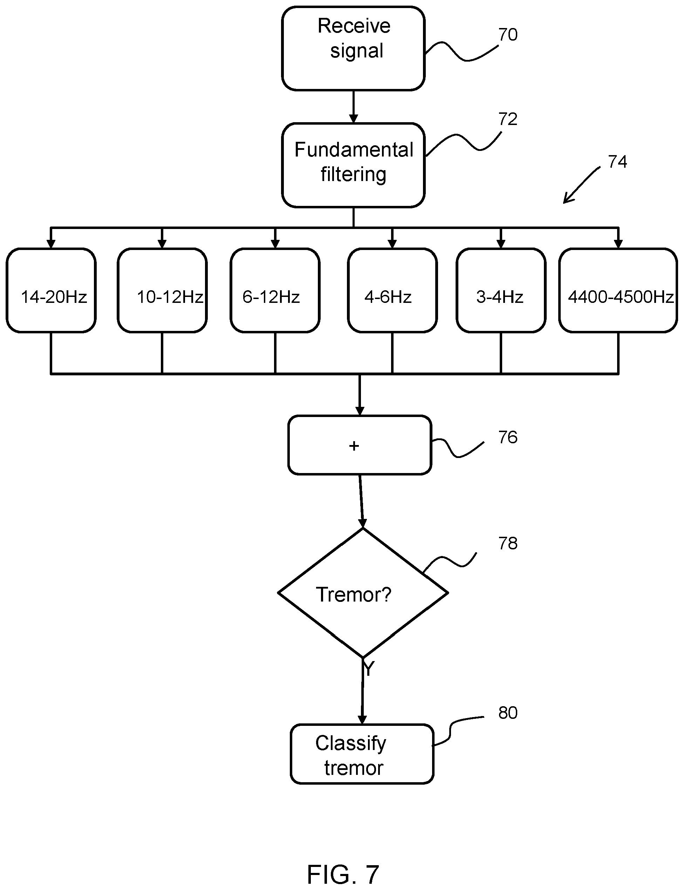

Fig. 7 shows a first example of a processing arrangement, to detect tremor vibrations from the sensor signal and thereby detect tremor, by filtering out characteristic frequency bands from the vibration of the toothbrush. A sonic and rotational toothbrush for example has sonic acoustic oscillations at 265 Hz and rotations at 70-80 Hz.

In step 70, the vibration signal is received. In step 72, the fundamental frequency of the toothbrush is filtered out. This is carried out as much as possible without reducing the specific tremor frequencies. In step 74 a set of narrowband filters is used, each designed for a frequency range associated with a particular type of tremor, within the overall tremor frequency range 3-20 Hz.

In the example shown, there is a 14 to 20Hz bandpass filter for orthostatic tremor, a 10 to 12Hz bandpass filter for physiologic tremor, a 6 to 12Hz bandpass filter for essential tremor, a 4 to 6Hz bandpass filter for Parkinsonism tremor, a 3 to 4Hz bandpass filter for cerebellar tremor, and a set of bandpass filters for harmonics detection are used in the range 4400Hz to 4500Hz.

The outputs of the filters are combined in step 76, and a threshold comparison in step 78 is used to classify whether or not a tremor is detected. If a tremor is detected, a comparison of the specific detected tremor frequencies can be done to classify the likely type of tremor detected in step 80.

The levels of the different filter outputs provide information about the severity of the tremor, if the specific amplitude of specific tremor frequency is higher for example. Changes over time can thereby also be tracked.

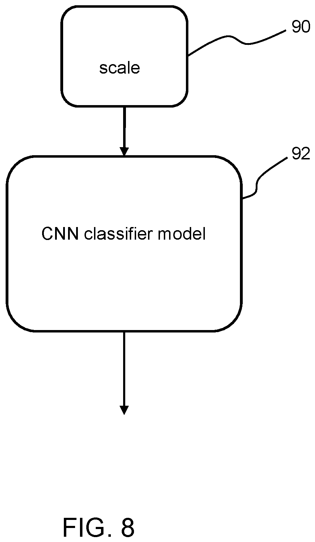

Fig. 8 shows a second example of a processing arrangement, to detect tremor vibrations from the sensor signal and thereby detect tremor, based on an AI approach. By way of example, a spectrogram image may be formed of a time window of vibration, such as a 1 second vibration signal sample, converted into an image. The image is then fed into a simple CNN based classifier model. Of course any suitable neural network may be employed.

In step 90, the spectrogram image is scaled, and it is fed into a CNN model 92, comprising an input layer and convolution layers. The AI model then generates a tremor classification.

The effectiveness of an AI based classification of tremor types has been tested, based on a spectrogram formed from each of a single axis accelerometer, a microphone and a motor current signal. A microphone may be considered to be an example of a single axis accelerometer, in that a single dimension motion signal is generated resulting from single axis movement of a microphone membrane. The motion of the personal care device is complex and multi-axis so the motion pattern on the single axis will change with orientation.

In addition to detecting the vibrations directly associated with a tremor, a person is suffering from any generalized motor disorder will have a disturbed brushing pattern. Using data acquired from a large data set it is also possible to train machine learning algorithms to detect typical brushing patterns and their deviation based on the sensor signal.

This information can be used to detect if a deviation in a detected brushing pattern is beyond certain acceptable threshold. This threshold can be defined based on a large data set of normal and physically limited persons' brushing patterns.

In the example above, the processor is shown as part of the toothbrush. Of course, the signal processing may be performed remotely, and the personal care device may transmit data to the remote processing system to perform the analysis and return the results. The signal processing analysis may be performed in real time or using data stored during the personal care (or health care) routine.

The invention has been described with reference to a toothbrush, but the same concept may be applied to any handheld device which is actuated such that it vibrates in use. The invention involves the detection of tremor based on the detectable interaction between the driven vibrations and the tremor vibrations.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner (optional)

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A hand-held personal care device comprising:
a handle (14);
an actuator (16) for implementing a personal care function which generates a vibration;
a sensor (20) for generating a sensor signal which depends on motion of the personal care device; and
a processing arrangement (22), configured to detect tremor vibrations from the sensor signal and thereby detect tremor,
wherein the sensor (20) comprises an actuator current sensor or a single axis vibration sensor.

2. The device of claim 1, wherein the sensor comprises a vibration sensor in the form of a microphone.

3. The device of claim 1 or 2, wherein the processing arrangement is configured to classify a detected tremor as one of a set of different types of tremor.

4. The device of any one of claims 1 to 3, wherein the processing arrangement is configured to determine a severity level of a detected tremor

5. The device of any one of claims 1 to 4, wherein the processing arrangement is configured to determine a progression of a detected tremor over time.

6. The device of any one of claims 1 to 5, wherein the processing arrangement comprises a band pass filter arrangement.

7. The device of claim 6, wherein the band pass filter arrangement comprises a first set of band pass filters within an expected frequency range of the tremors and a further band pass filter in a frequency range of higher harmonics of the expected frequency range of the tremors.

8. The device of claim 7, wherein the band pass filter arrangement comprises a further blocking filter for blocking an operating frequency of the vibration generated by the actuator.

9. The device of any one of claims 1 to 8, wherein the processing arrangement comprises a neural network.

10. The device of claim 9, wherein the neural network is supplied with a spectrogram of the sensor signal for a continuous range of frequencies up to at least double the personal care function vibration frequency.

11. The device of any one of claims 1 to 10 comprising a powered toothbrush

12. The device of any one of claims 1 to 11 comprising a shaver.

13. A method of detecting tremor, comprising:
receiving a sensor signal of an actuator current sensor or a single axis vibration sensor of a hand-held personal care device having an actuator for implementing a personal care function which generates a vibration; and
analyzing the sensor signal to detect tremor.

14. A computer program comprising computer program code means which is adapted, when said program is run on a computer, to implement the method of claim 13.
